# EUROPÄISCHE PATENTSCHRIFT

(11) **EP 3 011 326 B1**
(45) Veröffentlichungstag und Bekanntmachung des Hinweises auf die Patenterteilung: **05.09.2018**
(21) Anmeldenummer: 14727737.0
(22) Anmeldetag: 14.05.2014
(51) Int. Cl.: G01N 27/407, G01N 27/409, G01N 27/419, G01N 33/00

(54) **SENSORELEMENT MIT LEITERBAHN UND REFERENZGASKANAL**
SENSOR ELEMENT HAVING A PRINTED CONDUCTOR AND A REFERENCE GAS DUCT
ÉLÉMENT CAPTEUR DOTÉ D'UNE PISTE CONDUCTRICE ET D'UN CONDUIT DE GAZ DE REFERENCE

(30) Priorität: 21.06.2013 DE 102013211793
(43) Veröffentlichungstag der Anmeldung: 27.04.2016
(73) Patentinhaber: Robert Bosch GmbH, 70442 Stuttgart (DE)
(72) Erfinder: JUESTEL, Thomas, 96114 Hirschaid-Juliushof (DE); SCHNEIDER, Jens, 71229 Leonberg (DE); ROTTMANN, Andreas, 96188 Stettfeld (DE); BUSE, Frank, 70437 Stuttgart (DE)
(86) Internationale Anmeldenummer: PCT/EP2014/059833
(87) Internationale Veröffentlichungsnummer: WO 2014/202287

(56) Entgegenhaltungen:
- WO-A1-2011/153523
- DE-A1-102005 022 135
- DE-A1-102006 048 354
- DE-A1-102006 055 797
- DE-B4- 10 157 733
- JP-A- 2009 133 808
- US-A- 4 883 947
- US-A1- 2002 112 958
- US-A1- 2010 000 293

## Beschreibung

### Stand der Technik

Die Erfindung geht aus von bekannten Sensorelementen, welche beispielsweise als Abgassensoren eingesetzt werden, insbesondere als Lambdasonden, die in Kraftfahrzeugen eine sehr große Verbreitung gefunden haben. Auch auf andere Arten von Sensorelementen, ist die Erfindung jedoch anwendbar, zum Beispiel auf Sensoren zum Nachweis anderer gasförmiger Bestandteile von Abgasen und auf Partikelsensoren oder dergleichen.
Die Erfindung betrifft insbesondere ein gesintertes oder sinterbares keramisches Sensorelement, das beispielsweise durch Zusammenbringen, insbesondere Aufeinanderstapeln, einzelner, gegebenenfalls bedruckter, keramischer Grünfolien hergestellt ist.
Die Erfindung betrifft ferner insbesondere ein Sensorelement in dessen Innerem ein Referenzgaskanal ausgebildet ist.

Das Sensorelement umfasst ferner einen elektrischen Widerstandsheizer und eine Cermet-Elektrode als Funktionselemente in einem ersten, in der Regel dem Abgas zugewandten Endbereich des Sensorelements. Eine elektrische Versorgbarkeit des Sensorelements ist vorliegend durch Kontaktflächen auf der Außenfläche des Sensorelements in einem zweiten, in der Regel dem Abgas abgewandten, Bereich vorgesehen.

Eine elektrische Versorgbarkeit des Sensorelements erfolgt vorliegend durch elektrische Verbindungen der Funktionselemente mit den Kontaktflächen, die im Wesentlichen in Längsrichtung des Sensorelements im Inneren des Sensorelements verlaufende Leiterbahnen aufweisen. Zur Vermeidung von Sinterverzug und zur Optimierung der Wärmeleitung im Inneren des Sensorelements im Betrieb ist es attraktiv, die Leiterbahnen in Aufsicht auf das Sensorelement ganz oder teilweise (zum Beispiel mindestens 10% der Breite) überlappend mit dem Referenzgaskanal auszugestalten. Insbesondere die Auswirkungen der geringen Sinterschwindung und der geringen Wärmeleitfähigkeit eines ganz oder teilweise ungefüllten Referenzgaskanals lassen sich hierdurch bezogen auf das ganze Sensorelement kompensieren.
Problematisch ist, dass durch die vorgenannten Maßnahmen eine Kante des Referenzkanals mit den Leiterbahnen in Aufsicht auf das Sensorelement in Überdeckung kommt und somit im Sinne einer Schneidkante eine Verquetschung der Leiterbahnen in diesem Bereich während des Fertigungsprozesses potenziell verursachen kann.

Derartige Sensorelemente sind beispielsweise aus der DE 101 57 733 B4, der DE 10 2005 022 135 A1, der US 2002/0112958 A1 oder der DE 10 2006 048 354 A1 bekannt.

### Vorteile der Erfindung

Erfindungsgemäße Sensorelemente sind definiert durch die Merkmalen des Anspruchs 1. Sie haben gegenüber den bekannten Sensorelementen den Vorteil, dass der Bereich, in dem in Aufsicht auf das Sensorelement eine Kante des Referenzkanals mit den Leiterbahnen in Überdeckung kommt, in Längsrichtung des Sensorelements relativ weit erstreckt ist. Die potenzielle Schneidwirkung der Kante des Referenzgaskanals verteilt sich somit auf diese weite Erstreckung und eine Verquetschung der Leiterbahnen in diesem Bereich tritt resultierend in ungleich geringerem Maße in Erscheinung.

Hierfür ist erfindungsgemäß vorgesehen, dass die jeweilige Leiterbahn an ihrem dem ersten Endbereich des Sensorelements abgewandten Ende unter einem Winkel α von nicht mehr als 25°, insbesondere nicht mehr als 14°, zur Außenseite des Sensorelements 20° abgewinkelt verläuft.
Um den Bereich, in dem die Leiterbahn in Aufsicht auf Sensorelement ganz oder teilweise überlappend mit dem Referenzgaskanal ausgestaltetet ist, in seiner Länge und/oder Fläche nicht unnötig zu verringern, ist für den Winkel α auch eine Untergrenze vorgesehen, die er nicht unterschreiten darf, und die 2° oder 5° beträgt. Bevorzugt hat der Bereich, in dem die Leiterbahn abgewinkelt verläuft, eine Mindesterstreckung in Längsrichtung, die beispielsweise 2mm, 3mm oder sogar 4mm betragen kann oder durch die Breite der Leiterbahn gegeben sein kann.
Der durch die Abwinklung bewirkte seitliche Versatz des dem ersten Endbereich des Sensorelements abgewandten Endes der Leiterbahn ergibt sich insbesondere als Produkt der Erstreckung des Bereichs, in dem die Leiterbahn abgewinkelt verläuft, und dem inversen Tangens des Winkels a. Es ist bevorzugt, dass dieser seitliche Versatz nicht kleiner ist als die halbe oder volle Breite der Überdeckung zwischen der Leiterbahn und dem Referenzgaskanal. Der bevorzugte Versatz kann insbesondere auch nicht weniger als 0,3mm oder nicht weniger als 0,5mm betragen.
In speziellen Ausführungsformen der Erfindung ist der Referenzgaskanal ungefüllt, bildet also insbesondere einen mit Relation zu dem Sensorelement makroskopisch ausgebildeten Hohlraum mit beispielsweise rechteckigem Querschnitt. In diesem Fall ist zwar einerseits der Zutritt von Referenzluft zu dem Sensorelement grundsätzlich verbessert, die oben erläuterte Problematik der potenziellen Schneidwirkung der Kante des Referenzgaskanals ist jedoch zunächst noch verschärft. Erfindungsgemäß weist die jeweilige elektrisch leitende Verbindung zwischen dem Funktionselement und der Kontaktfläche neben der Leiterbahn, mit dieser zusammenwirkend jeweils eine Durchführung auf, die im Wesentlichen senkrecht zur Längsrichtung des Sensorelements verläuft. Die Durchführung besteht aus einer leitfähigen Beschichtung der radialen Wand eines Durchkontaktierlochs des Sensorelements. Der Referenzgaskanal ist in Aufsicht auf das Sensorelement mit der Durchführung überdeckungsfrei angeordnet, voraus der Vorteil resultiert, dass die Bruchfestigkeit des Sensorelements durch die Durchführung nur geringfügig vermindert ist.
Insofern von einer Leiterbahn die Rede ist, kann diese vorliegend eine Zuleitung und einen Kragen umfassen, der Kragen abgasabgewandt der Zuleitung angeordnet sein, die Zuleitung ganz oder zumindest in ihrem abgasabgewandten Teile eine konstante Breite aufweisen und/oder der Kragen ringförmig, zum Beispiel kreisringförmig, ausgebildet sein.

Das dem ersten Endbereich des Sensorelements abgewandte Ende der Leiterbahn kann insbesondere auch durch das dem ersten Endbereich des Sensorelements abgewandte Ende der Zuleitung und/oder durch die Gesamtheit aus dem dem ersten Endbereich des Sensorelements abgewandte Ende der Zuleitung zuzüglich dem Kragen der Leiterbahn gegeben sein.

Die Begriffe "Längsrichtung", "Querrichtung" und "Hochrichtung" werden im Rahmen dieser Anmeldung grundsätzlich lediglich im Sinne eines rechtwinkligen Bezugssystems verwendet. Insbesondere kann es sich jedoch überdies um Richtungen handeln, die durch das Sensorelement ausgezeichnet sind, beispielsweise kann bei einem insbesondere quaderförmigen Sensorelement die Längsrichtung die Richtung sein, in die die längsten Seitenkanten des Sensorelements weisen, die Hochrichtung die Richtung sein, in die die kürzesten Seitenkanten des Sensorelements weisen und/oder die Querrichtung die Richtung sein, in die die Seitenkanten des Sensorelements weisen, die eine mittlere Länge aufweisen. Beispielsweise kann bei einem stäbchenförmigen Sensorelement die Längsrichtung in Richtung einer Achse weisen, um die das stäbchenförmige Sensorelement rotationssymmetrisch bzw. im Wesentlichen rotationssymmetrisch ist.

Wo auf eine Richtung nur im Wesentlichen Bezug genommen wird, kommen neben der Richtung im engen Sinn auch Richtungen in Betracht, die von dieser Richtung geringfügig abweichen, zum Beispiel um nicht mehr als 15° und/oder Richtungen, die zu dieser Richtung zumindest nicht orthogonal sind. Eine Richtung ist durch eine Struktur zusätzlich auch dann im Wesentlichen realisiert, wenn die betreffende Struktur nur in einem kleinen Teilbereich, der zum Beispiel nicht mehr als 10% der Struktur umfasst, abgeweicht.

Unter "Länge des Sensorelements" wird die Erstreckung des Sensorelements in Längsrichtung, unter "Breite des Sensorelements" wird die Erstreckung des Sensorelements in Querrichtung und unter "Höhe des Sensorelements" wird die Erstreckung des Sensorelements in Hochrichtung im Rahmen dieser Anmeldung verstanden. Diese Richtung ist auch für die Aufsicht auf das Sensorelement maßgeblich.

Unter dem Begriff "Endbereich des Sensorelements" wird mit Bezug auf eine Längsrichtung im Rahmen dieser Anmeldung grundsätzlich lediglich ein zusammenhängender Teilbereich des Sensorelements verstanden, der das betreffende Ende des Sensors umfasst und nicht mehr als 50% der Länge des Sensorelements ausmacht. Insofern überschneiden sich ein Endbereich und ein gegenüberliegender Endbereich beispielsweise lediglich in einer Fläche. Etwas eingeschränkter kann ein Endbereich des Sensorelements insbesondere auch als ein zusammenhängender Teilbereich des Sensorelements verstanden werden, der das betreffende Ende des Sensors umfasst und nicht mehr als ein Drittel oder sogar nicht mehr als ein Viertel der Länge des Sensorelements ausmacht.

Der Begriff "Funktionselement" umfasst im Sinne der Erfindung eine mit dem Außenraum des Sensorelements kommunizierende Cermetelektrode und einen elektrischen Widerstandheizer, der insbesondere einen elektrischen Widerstand von maximal 30 Ohm bei 20°C aufweist. Im Fall des Widerstandheizers können die zwei Leiterbahnen der vorliegend beschriebenen Ausführungsformen nebeneinanderliegend, insbesondere spiegelsymmetrisch vorgesehen sein.

Im Fall der Cermetelektrode kann die Leiterbahn oder Zuleitung zu dieser Cermetelektrode dem Referenzgaskanal insbesondere unmittelbar gegenüberliegen. Aus diesem Grund kann es vorteilhaft sein, dass diese Leiterbahn oder Zuleitung in dem Bereich, in dem sie abgewinkelt verläuft, und/oder in dem Bereich in dem sie in Aufsicht auf das Sensorelement eine Kante des Referenzgaskanals schneidet, eine Breite aufweist, die gegenüber einem abgaszugewandten Bereich der Leiterbahn oder Zuleitung) erhöht ist, insbesondere um mindestens 25% und oder um mindestens 0,1mm.
Im Zusammenhang mit der vorliegenden Erfindung kann eine spezifische Materialwahl für Leiterbahnen, Zuleitungen, Durchführungen und Kontaktflächen weiterhin zielführend sein. Grundsätzlich sind hierbei Materialien mit einem Edelmetallanteil von 83 Gew.-% oder mehr bevorzugt, sodass vorgegebene ohmsche Widerstände mit minimiertem Edelmetalleinsatz erreicht werden können. Für zumindest eine Zuleitung zu der Heizvorrichtung sind sogar Edelmetallanteile von 95 Gew.-% oder mehr, zum Beispiel 98 Gew.-% bevorzugt. Ein Anteil von mindestens 1 Gew.-% Al2O3, besser sogar mindestens 1,5 Gew% AI2O3, bevorzugt maximal 2,5 Gew% AI2O3, hat sich zur präzisen Einstellbarkeit des elektrischen Widerstands dieser Strukturen als günstig herausgestellt.

Zumindest eine Zuleitung zu der Heizvorrichtung kann mit der Heizvorrichtung einstückig und aus dem gleichen Material ausgestaltet sein.

Zusätzlich oder alternativ ist für die Zuleitung zu der Cermetelektrode und/oder für zumindest eine Kontaktfläche ein geringerer Edelmetallanteil als für die zumindest eine Zuleitung zu der Heizvorrichtung vorgesehen, bevorzugt zum Beispiel 83 Gew.-% bis 87 Gew.-%, wobei insbesondere in der Zuleitung zu der Cermetelektrode ein Anteil an ZrO2 und Y2O3 von zusammen 12 Gew.-% bis 16 Gew.-% vorgesehen ist. Vorteil ist, dass die Zuleitung zu der Cermetelektrode zusammen mit der Cermetelektrode in einem Prozessschritt und aus dem gleichen Material hergestellt werden kann. Auch für die Zuleitung zu der Cermetelektrode bzw. für die Cermetelektrode ist ein Al2O3-Anteil, bevorzugt 0,2 Gew.-% bis 1 Gew.-% vorteilhaft.

Zusätzlich oder alternativ ist für zumindest eine Durchführung ein geringerer Edelmetallanteil als für die zumindest eine Zuleitung zu der Heizvorrichtung vorgesehen, bevorzugt zum Beispiel 83 Gew.-% bis 87 Gew.-%, wobei in der Durchführung ein Anteil an ZrO2 und Y2O3 von zusammen 3 Gew.-% bis 8 Gew.-% und zusätzlich ein Anteil von Nb2O5 von 6 Gew.-% bis 12 Gew.-% vorgesehen ist. Vorteil ist, dass die Durchführungen im Fertigungsprozess besser handhabbar sind. Insbesondere weisen entsprechende Pasten bessere rheologische Eigenschaften auf und ermöglichen eine bessere keramische Anbindung der Durchführungen innerhalb der Sensorelemente. Im Zusammenhang mit Sensorelementen, die überwiegend aus YSZ bestehen, bildet sich so überdies in den Randbereichen der Durchführungen eine verminderte Sauerstoffionenleitfähigkeit aus, was die Funktionalität der Sensorelemente verbessert.

Die oben genannten Edelmetallanteile können insbesondere aus Platin bestehen. Alternativ, insbesondere in Bezug auf zumindest eine Durchführung, können zur Stabilisierung der Metallphase Anteile, bevorzugt 0,2 Gew.-% bis 0,8 Gew.-% bezogen auf die Gesamtzusammensetzung der Materialien, aus Rhodium bestehen und/oder Anteile, bevorzugt 0,2 Gew.-% bis 1 Gew.-% bezogen auf die Gesamtzusammensetzung der Materialien, aus Palladium bestehen.

Weitere Edelmetallanteile können stets vorgesehen sein.

### Zeichnung

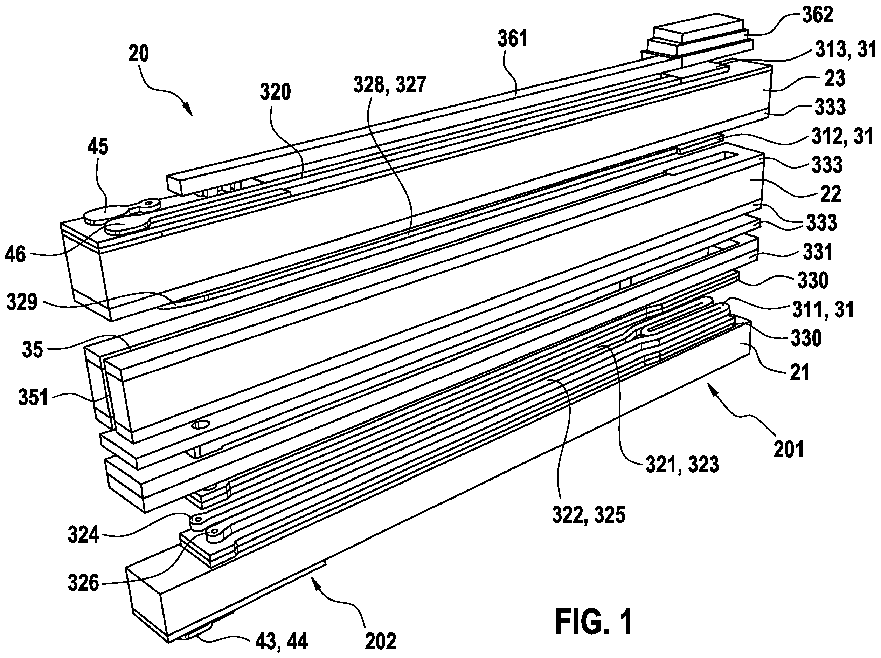

Die Figur 1 zeigt in Explosionsdarstellung perspektivisch und schematisch ein erfindungsgemäßes Sensorelement

Die weiteren Figuren zeigen vergrößerte Detailansichten

### Beschreibung des Ausführungsbeispiels

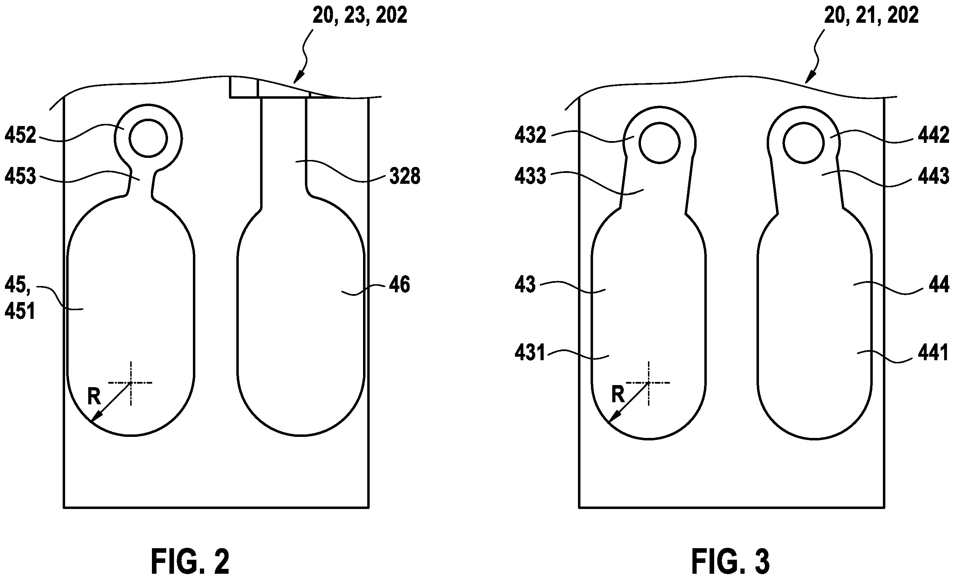

Figur 1 zeigt als ein Ausführungsbeispiel der Erfindung eine Gesamtansicht eines Sensorelements 20, das in einem Gehäuse eines Gasmessfühlers (nicht gezeichnet) angeordnet werden kann, der zur Bestimmung der Sauerstoffkonzentration in einem Abgas eines Verbrennungsmotors (nicht gezeichnet) dient. Mit entsprechenden Funktionselementen versehen ist die Erfindung selbstverständlich auch als Sensorelement für andere Sensoren, beispielsweise Sensoren zur Partikelmessung geeignet.
Das Sensorelement erstreckt sich in der Figur 1 in Längsrichtung von links nach rechts, wobei ein erster Endbereich 201 des Sensorelements 20 rechts und ein zweiter Endbereich 202 des Sensorelements 20 links abgebildet ist. Im bestimmungsgemäßen Verbau und Betrieb ist der erste Endbereich 201 des Sensorelements 20 einem Abgas zugewandt und der zweite Endbereich 202 des Sensorelements 20 dem Abgas abgewandt.
Ferner erstreckt sich in der Figur 1 das Sensorelement 20 in Querrichtung von vorne nach hinten und in Hochrichtung von unten nach oben.
Das Sensorelement 20 ist aus bedruckten keramischen Schichten aufgebaut, die in diesem Beispiel als eine erste, zweite und eine dritte Festelektrolytfolie 21, 22, 23 ausgebildet sind und Yttriumoxid stabilisiertes Zirkonoxid (YSZ) enthalten. Die Festelektrolytfolien 21, 22, 23 weisen im Beispiel vor einem Sintervorgang eine Länge von 72mm, eine Breite von 5mm und eine Höhe von 540µm auf. Folien eines gesinterten Sensorelements 20 weisen um 20% verminderte Kantenlängen auf.
Die erste Festelektrolytfolie 21 ist auf ihrer aus Sicht des Sensorelements 20 nach außen weisenden Großfläche, in Figur 1 unten, im zweiten Endbereich 202 des Sensorelements 20 mit einer Kontaktfläche 43 und einer weiteren Kontaktfläche 44 versehen, hier bedruckt; siehe auch Figur 3.

Die erste Festelektrolytfolie 21 ist auf ihrer aus Sicht des Sensorelements 20 nach innen weisenden Großfläche, in Figur 1 oben, im ersten Endbereich 201 des Sensorelements 20 mit einer mäanderförmigen Heizvorrichtung 311 als ein Funktionselement 31, das der Beheizung des ersten Endbereichs 201 des Sensorelements 20 dient, versehen. In Fortsetzung der mäanderförmigen Heizvorrichtung 311 ist an deren Enden jeweils eine Leiterbahn 321, 322 angeschlossen, wobei der Übergang von Heizvorrichtung 311 zu Leiterbahn 321, 322 durch eine Zunahme der Strukturbreite und/oder -höhe bzw. eine Abnahme des elektrischen Widerstandes pro Länge gekennzeichnet ist.

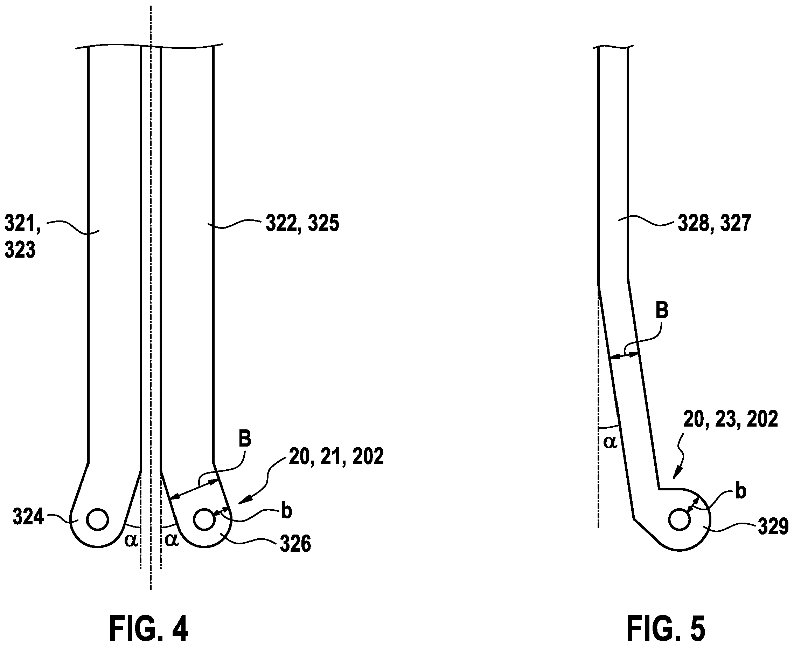

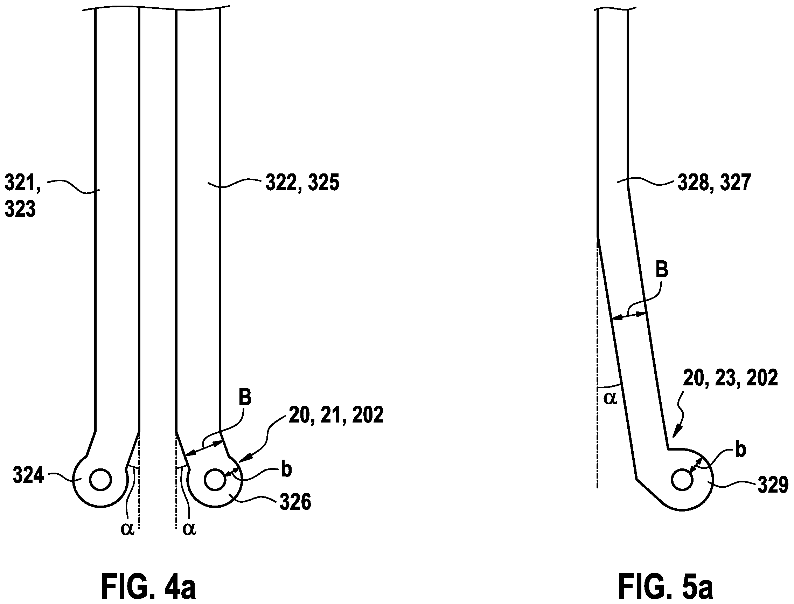

Die Leiterbahnen 321, 322 weisen abgasseitig einen als Zuleitung 323, 325 bezeichneten Abschnitt auf, der vorliegend eine konstante Breite hat. Die Leiterbahnen 321, 322 weisen ferner abgasabgewandt einen als Kragen 324, 326 bezeichneten Abschnitt, der vorliegend ringförmig ausgebildet ist, auf; siehe auch Figur 4.

Die erste Festelektrolytfolie 21 ist auf ihrer aus Sicht des Sensorelements 20 nach innen weisenden Großfläche, in Figur 1 oben, ferner mit Isolationsschichten 330 und einem Dichtrahmen 331, sowie einer Folienbinderschicht 333 versehen, hier bedruckt.

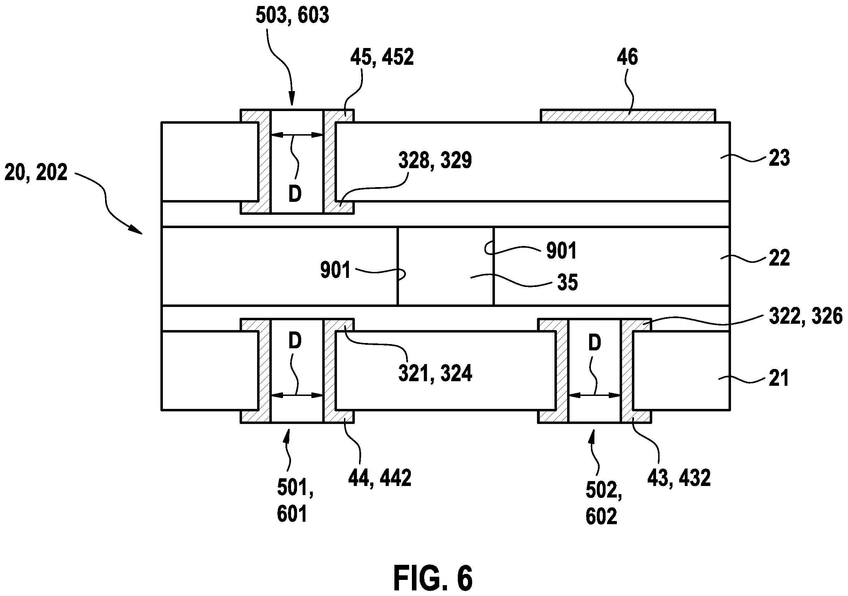

Die erste Festelektrolytfolie 21 weist im zweiten Endbereich 202 zwei Durchführungen 501, 502 auf, die in senkrechter Richtung durch die erste Festelektrolytfolie 21 verlaufen und jeweils eine Kontaktfläche 43, 44 mit einem Kragen 324, 326 einer Leiterbahn 321, 322 elektrisch leitendend verbinden; siehe Figur 6.

Die zweite Festelektrolytfolie 22 ist beidseitig mit jeweils einer Folienbinderschicht 333 versehen, ferner weist die zweite Festelektrolytfolie 22 einen Referenzgaskanal 35 auf, der sich längs von einer abgasabgewandt angeordneten Referenzgasöffnung 351 bis in den ersten Endbereich 201 des Sensorelements 20 erstreckt und dabei in Querrichtung mittig verläuft. Der Referenzgaskanal 35 ist ungefüllt ausgebildet, insbesondere sind in ihm keine porösen Füllungen vorgesehen.

Die dritte Festelektrolytfolie 23 ist auf ihrer aus Sicht des Sensorelements 20 nach innen weisenden Großfläche, in Figur 1 unten, dem Referenzgaskanal 35 gegenüberliegend, mit einer Cermetelektrode 312 als Funktionselement 31 zur Messung einer Sauerstoffkonzentration versehen. In Fortsetzung der Cermetelektrode 312 ist an deren Ende eine Leiterbahn 328 angeschlossen, wobei der Übergang von der Cermetelektrode zu der Leiterbahn 328 durch eine Abnahme der Strukturbreite gekennzeichnet ist.

Die Leiterbahn 328 weist abgasseitig einen als Zuleitung 327 bezeichneten Abschnitt auf, der vorliegend eine konstante Breite hat. Die Leiterbahn 328 weist ferner abgasabgewandt einen als Kragen 329 bezeichneten Abschnitt, der vorliegend ringförmig ausgebildet ist, auf; siehe auch Figur 5. Auf dieser Seite der dritten Festelektrolytschicht 23 ist, zumindest wo ansonsten unbedruckt, eine Folienbinderschicht 333 vorgesehen.

Die dritte Festelektrolytfolie 23 ist auf ihrer aus Sicht des Sensorelements 20 nach außen weisenden Großfläche, in Figur 1 oben, im zweiten Endbereich 202 des Sensorelements 20 mit einer Kontaktfläche 45 und einer weiteren Kontaktfläche 46 versehen, hier bedruckt; siehe auch Figur 2.

An die weitere Kontaktfläche 46 schließt sich eine Leiterbahn 320 mit beispielsweise konstanter Breite an, die sich bis zu einer im ersten Endebereich 201 des Sensorelements 20 angeordneten weiteren Cermetelektrode 313 erstreckt. Die Leiterbahn 320 ist mit einer zum Beispiel dichten Abdeckschicht 361 bedeckt, die weitere Cermetelektrode 313 ist mit porösen Schichten 362 versehen, sodass eine Kommunikation zwischen Außenraum und weiterer Cermetelektrode 313 gewährleistet ist.

Die dritte Festelektrolytfolie 23 weist im zweiten Endereich eine Durchführung 503 auf, die die in senkrechter Richtung durch die dritte Festelektrolytfolie 23 verläuft und die die Kontaktfläche 45 mit dem Kragen 329 elektrisch leitendend verbindet; siehe Figur 6.

In der Figur 2 ist der zweite, abgasabgewandte Endbereich 202 des Sensorelements 20 in Aufsicht auf die dritte Festelektrolytfolie 23 gezeigt. Dort ist mit Blick zum ersten, abgaszugewandten Endbereich 201 des Sensorelements 20 die Kontaktfläche 45 links angeordnet.

Die Kontaktfläche 45 setzt sich zusammen aus drei Teilbereichen, nämlich einem Rumpfbereich 451, einem Kopfbereich 452 und einem Halsbereich 453. Der Rumpfbereich 451 ist auf der dem Abgas abgewandten Seite der Kontaktfläche 45 angeordnet. Er hat eine längliche Grundform, die aus einem Rechteck gleicher Länge und Breite durch maximale Abrundung der Ecken hervorgeht, also durch eine Abrundung mit einem Krümmungsradius R, der der halben Breite des Rumpfbereichs 451 bzw. der Kontaktfläche 45 entspricht. Auf diese Weise entstehen somit halbkreisförmige Endbereiche des Rumpfbereiches 451 bzw. der Kontaktfläche 45 auf der dem Abgas abgewandten Seite der Kontaktfläche 45.

Bezogen auf ein ungesintertes Sensorelement 20 (gesintert: -20%) beträgt die Länge des Rumpfbereiches 451 in diesem Beispiel 2,5mm oder mehr, die Breite des Rumpfbereichs 4511,5mm oder mehr. Der Rumpfbereich 451 ist von der linken Außenkante des Sensorelements 20 0,4mm oder weniger beabstandet und von der vorderen Außenkante des Sensorelements 20 1,3mm oder weniger beabstandet.

Der Kopfbereich 452 ist auf der dem Abgas zugewandten Seite der Kontaktfläche 45 angeordnet. Der Kopfbereich 452 ist beispielsweise ringförmig ausgebildet, mit einem Innendurchmesser von 0,5mm oder weniger und einem Außendurchmesser von 1mm oder mehr, bezogen auf ein ungesintertes Sensorelement 20 (gesintert: -20%).

Der Halsbereich 453 ist zwischen dem Rumpfbereich 451 und dem Kopfbereich 452 ausgebildet. Er bildet gegenüber dem Rumpfbereich 451 und dem Kopfbereich 452 eine Einschnürung der Kontaktfläche 45 mit einer minimalen Breite von im Beispiel 0,3mm und einer Länge von 0,3mm, bezogen auf ein ungesintertes Sensorelement 20 (gesintert: - 20%).

Der Rumpfbereich 451 weist im Beispiel eine Spiegelsymmetrie bezüglich einer Achse auf, die in Längsrichtung des Sensorelements 20 weist. Der Kopfbereich 452 und der Halsbereich 453 weisen ebenfalls eine Spiegelsymmetrie auf, allerdings bezüglich einer Achse, die gegenüber der Längsachse des Sensorelements 20 um 9° bei Aufsicht auf das Sensorelement 20 im mathematisch negativen Drehsinn verdreht ist, sodass der Kopfbereich 452 und der Halsbereich 453 insgesamt leicht zur Sensormitte hin verkippt sind.

Der Kopfbereich 452 der Kontaktfläche 45 wirkt mit einer Durchführung 503 durch die dritte Festelektrolytschicht 23 elektrisch leitend zusammen.

In Figur 2 ist überdies mit Blick zum ersten, abgaszugewandte Endbereich 201 des Sensorelements 20 die weitere Kontaktfläche 46 rechts neben der Kontaktfläche 45 angeordnet. Anordnung und Größe der weiteren Kontaktfläche 46 entspricht in diesem Sinn, also unter Vertauschung von links und rechts, der Anordnung und der Größe des Rumpfbereichs 451 der Kontaktfläche 45 mit der Maßgabe, dass zwischen der Kontaktfläche 45 und der weiteren Kontaktfläche 46 ein Abstand von mindestens 0,6mm besteht, bezogen auf ein ungesintertes Sensorelement 20 (gesintert: -20%).

Die weitere Kontaktfläche 46 besteht lediglich aus einem dem Rumpfbereich 451 der Kontaktfläche 45 entsprechenden Teil, weist also weder Kopf- noch Halsbereich auf. Er wirkt auch nicht mit einer Durchführung zusammen, stattdessen ist er unmittelbar mit der Leiterbahn 328 kontaktiert, die zu der weiteren Cermetelektrode 313 führt. Eine Mittenachse der Leiterbahn 328 in Längsrichtung ist dabei bezogen auf eine Mittenachse der weiteren Kontaktfläche 46 in Längsrichtung um 0,1mm bis 0,4mm, im Beispiel um 0,2mm, quer nach innen verschoben, bezogen auf ein ungesintertes Sensorelement 20 (gesintert: -20%).

Die Kontaktflächen 45, 46 weisen einen Edelmetallanteil von 83 Gew.-% bis 87 Gew.-%, und einen Anteil an ZrO2 und von zusammen 12 Gew.-% bis 16 Gew.-% auf.

In die Figur 3 ist der zweite, abgasabgewandte Endbereich 202 des Sensorelements 20 in Untersicht unter die in Figur 1 nach unten weisende erste Festelektrolytfolie 21 gezeigt. Dort ist mit Blick zum ersten, abgaszugewandte Endbereich 201 des Sensorelements 20 die Kontaktfläche 43 links angeordnet.

Die Kontaktfläche 43 setzt sich zusammen aus drei Teilbereichen, nämlich einem Rumpfbereich 431, einem Kopfbereich 432 und einem Halsbereich 433. Der Rumpfbereich 431 ist auf der dem Abgas abgewandten Seite der Kontaktfläche 43 angeordnet. Er hat eine längliche Grundform, die aus einem Rechteck gleicher Länge und Breite durch maximale Abrundung der Ecken hervorgeht, also durch eine Abrundung mit einem Krümmungsradius R, der der halben Breite des Rumpfbereichs 431 bzw. der Kontaktfläche 43 entspricht. Auf diese Weise entstehen somit halbkreisförmige Endbereiche des Rumpfbereiches 431 bzw. der Kontaktfläche 43 auf der dem Abgas abgewandten Seite der Kontaktfläche 43.

Bezogen auf ein ungesintertes Sensorelement 20 (gesintert: -20%) beträgt die Länge des Rumpfbereiches 431 in diesem Beispiel 2,5mm oder mehr, die Breite des Rumpfbereichs 4311,5mm oder mehr. Der Rumpfbereich 431 ist von der linken Außenkante des Sensorelements 20 0,4mm oder weniger beabstandet und von der vorderen Außenkante des Sensorelements 20 1,3mm oder weniger beabstandet.

Der Kopfbereich 432 ist auf der dem Abgas zugewandten Seite der Kontaktfläche 43 angeordnet. Der Kopfbereich 432 ist beispielsweise ringförmig ausgebildet, mit einem Innendurchmesser von 0,5mm oder weniger und einem Außendurchmesser von 1mm oder mehr, bezogen auf ein ungesintertes Sensorelement 20 (gesintert: -20%).

Der Halsbereich 433 ist zwischen dem Rumpfbereich 431 und dem Kopfbereich 432 ausgebildet. Er bildet gegenüber dem Rumpfbereich 431 und dem Kopfbereich 432 eine Einschnürung der Kontaktfläche 43 mit einer minimalen Breite von im Beispiel 0,9mm und einer Länge von 0,3mm, bezogen auf ein ungesintertes Sensorelement 20 (gesintert: - 20%).

Der Halsbereich 433 der Kontaktfläche 43 ist wesentlich breiter, hier um einen Faktor größer 2, als der Halsbereich 451 der Kontaktfläche 45 in Figur 2. Hintergrund ist, dass über die Kontaktfläche 43 der Heizvorrichtung 311 hohe Ströme zugeführt werden, während über die Kontaktfläche 45 der Cermetelektrode 312 nur vergleichsweise geringe Ströme zugeführt werden. Die Kontaktfläche 43 ist folglich mit einem verminderten ohmschen Widerstand bzw. verbreitertem Halsbereich 433 ausgestaltet.

Der Rumpfbereich 431 weist im Beispiel eine Spiegelsymmetrie auf, bezüglich einer Achse, die in Längsrichtung des Sensorelements 20 weist. Der Kopfbereich 432 und der Halsbereich 433 weisen ebenfalls eine Spielgelsymmetrie auf, allerdings bezüglich einer Achse, die gegenüber der Längsachse des Sensorelements 20 um 9° bei Aufsicht auf das Sensorelement 20 im mathematisch negativen Drehsinn verdreht ist, sodass der Kopfbereich 432 und der Halsbereich 433 insgesamt leicht zur Sensormitte hin verkippt sind.

Der Kopfbereich 432 der Kontaktfläche 43 wirkt mit einer Durchführung 501 durch die erste Festelektrolytschicht 21 elektrisch leitend zusammen.

In Figur 3 ist überdies mit Blick zum ersten, abgaszugewandten Endbereich 201 des Sensorelements 20 die weitere Kontaktfläche 44 rechts neben der Kontaktfläche 43 angeordnet. Anordnung und Größe der weiteren Kontaktfläche 46 entsprechen in diesem Sinn, also unter Vertauschung von links und rechts und von positivem Drehsinn mit negativen Drehsinn, der Anordnung und der Größe der Kontaktfläche 43 mit der Maßgabe, dass zwischen der Kontaktfläche 43 und der weiteren Kontaktfläche 44 ein Abstand von mindestens 0,6mm besteht, bezogen auf ein ungesintertes Sensorelement 20 (gesintert: -20%).

Die Kontaktflächen 43, 44 weisen einen Edelmetallanteil von 83 Gew.-% bis 87 Gew.-%, und einen Anteil an ZrO2 und von zusammen 12 Gew.-% bis 16 Gew.-% auf.

In der Figur 4 ist der zweite, abgasabgewandte Endbereich 202 des Sensorelements 20 in Aufsicht auf die erste Festelektrolytfolie 21, in Figur 1 von oben, gezeigt. Dort ist mit Blick zum ersten, abgaszugewandten Endbereich 201 des Sensorelements 20 die Leiterbahn 322 rechts angeordnet. Die Leiterbahn 322 setzt sich zusammen aus zwei Teilbereichen, nämlich einer Zuleitung 325 und einem Kragen 326.

Die Zuleitung 325 bildet den abgasseitigen Teil der Leiterbahn 322 und erstreckt sich von der Heizvorrichtung 311 abgasseitig bis zu dem abgasabgewandt der Zuleitung 325 angeordneten Kragen 326. Vorliegend weist die Zuleitung 325 eine Breite B von 1,2mm auf und verläuft abgasseitig mit einer Beabstandung in Querrichtung von 0,25mm zur mittleren Längsachse des Sensorelements 20, jeweils bezogen auf ein ungesintertes Sensorelement 20 (gesintert: -20%). In einem abgasabgewandten Endbereich ist die Zuleitung 325 nach rechts, also nach außen, unter einem Winkel von 18° abgewinkelt.

Der Kragen 326 ist ringförmig ausgebildet und beschreibt vorliegend einen Bogen von 180°, dessen Außendurchmesser mit der Breite B der Zuleitung 325 identisch ist und dessen Innendurchmesser 0,4mm beträgt. Eine Breite des Kragens beträgt somit 0,3mm, jeweils bezogen auf ein ungesintertes Sensorelement 20 (gesintert: -20%). Ein Breitenverhältnis aus Kragenbreite b zu Zuleitungsbreite B beträgt 0,33.

Der elektrische Widerstand der Durchführung 501 ist dem elektrischen Widerstand der Leiterbahn 322 gleich oder etwa gleich, bezogen auf eine Temperaturverteilung, die im Betrieb des Sensors auftreten bzw. typischerweise auftreten kann. Neben einer homogenen Temperaturverteilung, zum Beispiel 20°C, ist hierbei alternativ auch auf Temperaturverteilungen abstellbar, die inhomogen sind. Beispielsweise können gleichmäßige Temperaturanstiege in Längsrichtung von 1100°C im Bereich der Heizvorrichtung 311 und 200°C, 300°C oder sogar 400°C im Bereich der Durchführung 501 zugrunde gelegt werden.

Der elektrische Widerstand der elektrischen Verbindung des Funktionselements, insbesondere der Heizvorrichtung 311 mit der Kontaktfläche 43 liegt beispielsweise im Bereich von 2,5 Ohm bei 20°C.

In Figur 4 ist überdies mit Blick zum ersten, abgaszugewandte Endbereich 201 des Sensorelements 20 die Leiterbahn 321 bezogen auf die mittlere Längsachse zu der Leiterbahn 322 symmetrisch angeordnet. Anordnung und Größe der Leiterbahn 321 entspricht in diesem Sinn, also unter Vertauschung von links und rechts, der Anordnung und der Größe der Leiterbahn 322.

Die Zuleitungen 325, 323 weisen einen Edelmetallanteil von mehr als 95 Gew.-% zum Beispiel 98 Gew.-% und mindestens 1 Gew.-% Al2O3 auf.

Der elektrische Widerstand der Durchführung 502 ist dem elektrischen Widerstand der Leiterbahn 321 gleich oder etwa gleich, bezogen auf eine Temperaturverteilung, die im Betrieb des Sensors auftreten bzw. typischerweise auftreten kann. Neben einer homogenen Temperaturverteilung, zum Beispiel 20°C, ist hierbei alternativ auch auf Temperaturverteilungen abstellbar, die inhomogen sind. Beispielsweise können gleichmäßige Temperaturanstiege in Längsrichtung von 1100°C im Bereich des Heizvorrichtung 311 und 200°C, 300°C oder sogar 400°C im Bereich der Durchführung 501 zugrunde gelegt werden

Die Figur 4a zeigt als Variante ein Sensorelement 20 mit geringfügig modifizierten Zuleitungen 323, 325, wobei die Modifikation lediglich darin besteht, dass die Breite B der Zuleitungen 323, 225 nur 1,08mm statt 1,2mm beträgt, also im Vergleich zum Kragen 324, 326 geringfügig (10%) reduziert ist. Die metrischen Maße sind bezogen auf ein ungesintertes Sensorelement 20 (gesintert -20%).

In der Figur 5 ist der zweite, abgasabgewandte Endbereich 202 des Sensorelements 20 in Untersicht unter die dritte Festelektrolytfolie 23, in Figur 3 von unten, gezeigt. Dort ist mit Blick zum ersten, abgaszugewandte Endbereich 201 des Sensorelements 20 die Leiterbahn 322 rechts angeordnet. Die Leiterbahn 322 setzt sich zusammen aus zwei Teilbereichen, nämlich einer Zuleitung 327 und einem Kragen 329.

Die Zuleitung 327 bildet den abgasseitigen Teil der Leiterbahn und erstreckt sich von der Cermetelektrode 312 abgasseitig bis zu dem abgasabgewandt der Zuleitung 327 angeordneten Kragen 329. Vorliegend weist die Zuleitung eine Breite B von 0,4mm (ungesintert; gesintert: -20%) auf und verläuft abgasseitig so, dass sie, in senkrechter Projektion in Aufsicht auf das Sensorelement 20, innerhalb des Referenzgaskanals 35 angeordnet ist. Dieser Teil der Zuleitung 327 ist während des Fertigungsprozess somit weitgehend vor Verquetschungen geschützt.

In einem abgasabgewandten Endbereich ist die Zuleitung 327 nach rechts, also nach außen, unter einem Winkel von nicht mehr als 25°, hier von 8°, abgewinkelt. In diesem abgasabgewandten Endbereich schneidet sich die Zuleitung mit der Kante des Referenzgaskanals 35 in senkrechter Projektion in Aufsicht auf das Sensorelement 20. Durch den vergleichsweise kleinen Schnittwinkel ergibt sich eine lange Überlappungszone zwischen Leiterbahn 328 und Kante des Referenzgaskanals 35 und somit wiederum ein guter Schutz vor Verquetschungen der Zuleitung 327 während des Fertigungsprozesses.

Der Kragen 329 ist ringförmig ausgebildet. Eine Breite des Kragens b beträgt 0,3mm, bezogen auf ein ungesintertes Sensorelement 20 (gesintert: -20%). Ein Breitenverhältnis aus Kragenbreite b zu Zuleitungsbreite B beträgt 0,75.

Die Zuleitung 327 weist einen Edelmetallanteil von 83 Gew.-% bis 87 Gew.-%, und einen Anteil an ZrO2 und von zusammen 12 Gew.-% bis 16 Gew.-% auf.

Der elektrische Widerstand der Durchführung 503 ist dem elektrischen Widerstand der Leiterbahn 328 gleich oder etwa gleich, bezogen auf eine Temperaturverteilung, die im Betrieb des Sensors auftreten bzw. typischerweise auftreten kann. Neben einer homogenen Temperaturverteilung, zum Beispiel 20°C, ist hierbei alternativ auch auf Temperaturverteilungen abstellbar, die inhomogen sind. Beispielsweise können gleichmäßige Temperaturanstiege in Längsrichtung von 750°C im Bereich der Cermetelektrode 312 und 200°C, 300°C oder sogar 400°C im Bereich der Durchführung 503 zugrunde gelegt werden.

Die Figur 5a zeigt als Variante ein Sensorelement 20 mit geringfügig modifizierter Zuleitung 328, wobei die Modifikation lediglich darin besteht, dass die Breite B der Zuleitung 328 im abgasabgewandten Endbereich gegenüber dem abgaszugewandten Bereich der Zuleitung 328 um 50% erhöht ist, von 0,4mm auf 0,6mm. Die metrischen Maße sind bezogen auf ein ungesintertes Sensorelement 20 (gesintert -20%).

Ein Schnitt durch das in den vorangehende Figuren 1 bis 5 gezeigte Sensorelement 20, in einer Ebene senkrecht zur Längsrichtung des Sensorelements 20 durch die Durchführungen 501, 502, 503 ist in der Figur 6, rein schematisch, gezeigt.

Die Durchführungen 501, 502, 503 sind als leitfähige Beschichtung der radialen Wand eines Durchkontaktierlochs 601, 602, 603 des Sensorelements 20 ausgebildet. Der Durchmesser der Durchkontaktierlöcher 601, 602, 603 beträgt im Beispiel 0,6mm, bezogen auf ein ungesintertes Sensorelement 20 (gesintert: -20%, also 0,48mm).

Ersichtlich sind die Durchführungen 501, 502, 503 jeweils mit dem Referenzgaskanal 35 in Aufsicht auf das Sensorelement 20 überdeckungsfrei ausgeführt.

Die Durchführungen 501, 502, 503 weisen einen Edelmetallanteil von 83 Gew.-% bis 87 Gew.-%, und einen Anteil an ZrO2 und von zusammen 3 Gew.-% bis 8 Gew.-% und zusätzlich ein Anteil von Nb2O5 von 6 Gew.-% bis 12 Gew.-% auf.

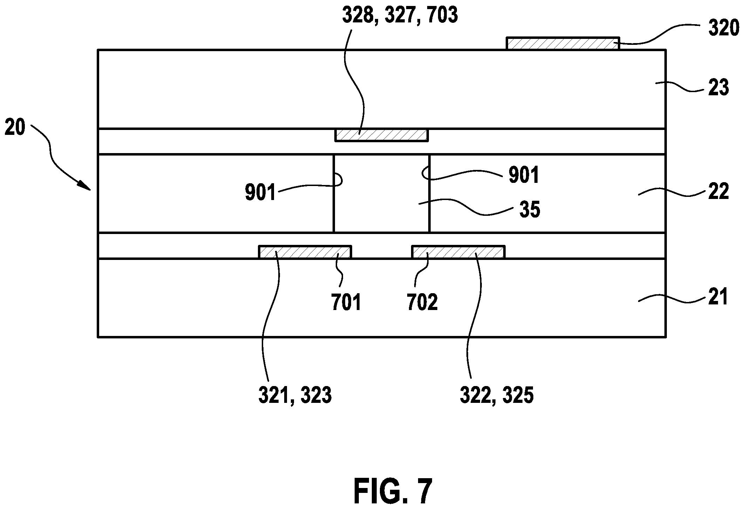

Ein Schnitt durch das in den vorangehende Figuren 1 bis 5 gezeigte Sensorelement 20, in einer Ebene senkrecht zur Längsrichtung des Sensorelements 20 etwa im Bereich der halben Längserstreckung des Sensorelements 20 ist in der Figur 7, rein schematisch, gezeigt.

Wie ersichtlich, kommt in Aufsicht auf das Sensorelement 20 die Leiterbahn 328 bzw. die Zuleitung 327, die zu der Cermetelektrode 312 führen, über ihre volle Breite mit dem Referenzkanal 35 zur Überdeckung 703. Ferner kommen die Leiterbahnen 321, 322, bzw. die Zuleitungen 323, 325, die zu dem Widerstandsheizer führen, über jeweils ca. 10% ihrer Breite mit dem Referenzkanal 35 zur Überdeckung 701, 702.

## Patentansprüche

1. Sensorelement, insbesondere zum Nachweis einer physikalischen Eigenschaft eines Gases, insbesondere zum Nachweis der Konzentration einer Gaskomponente oder der Temperatur oder eines festen Bestandteils oder eines flüssigen Bestandteils eines Abgases eines Verbrennungsmotors, wobei das Sensorelement (20) in seiner Längsrichtung einander gegenüberliegend einen ersten Endbereich (201) und einen zweiten Endbereich (202) aufweist, wobei das Sensorelement (20) in dem ersten Endbereich (201), im Inneren des Sensorelements (20) einen elektrischen Widerstandheizer (311) aufweist, der mit einer im zweiten Endbereich (202) auf der Außenfläche des Sensorelements (20) angeordneten ersten Kontaktfläche (43) elektrisch leitend verbunden ist, wobei die elektrisch leitende Verbindung zwischen dem Widerstandsheizer (311) und der ersten Kontaktfläche (43) eine im Inneren des Sensorelements (20) im Wesentlichen in Längsrichtung verlaufende erste Leiterbahn (321) aufweist, wobei die elektrisch leitende Verbindung zwischen dem Widerstandsheizer (311) und der ersten Kontaktfläche (43) neben der ersten Leiterbahn (321), mit dieser zusammenwirkend eine erste Durchführung (501) aufweist, die im Wesentlichen senkrecht zur Längsrichtung des Sensorelements (20) verläuft, wobei die erste Durchführung (501) aus einer leitfähigen Beschichtung der radialen Wand eines ersten Durchkontaktierlochs (601) des Sensorelements (20) besteht, wobei das Sensorelement (20) ferner einen im Wesentlichen in Längsrichtung des Sensorelements (20) verlaufenden über eine Referenzgasöffnung (351) mit einem sich außerhalb des Sensorelements (20) befindlichen Referenzgas kommunizierenden Referenzgaskanal (35) aufweist, wobei der Referenzgaskanal (35) in Aufsicht auf das Sensorelement (20) mit der ersten Durchführung (501) überdeckungsfrei angeordnet ist, wobei die erste Leiterbahn (321) und der Referenzgaskanal (35) so angeordnet sind, dass es in Aufsicht auf das Sensorelement (20) zwischen ihnen zu einer ersten zumindest teilweisen Überdeckung (701) kommt, wobei die erste Leiterbahn (321) an ihrem dem ersten Endbereich (201) des Sensorelements (20) abgewandten Ende unter einem Winkel (α) von nicht mehr als 25°, insbesondere nicht mehr als 14°, und nicht weniger als 2°, insbesondere nicht weniger als 5°, zur Außenseite des Sensorelements (20) abgewinkelt verläuft und wobei der Widerstandsheizer (311) mit einer im zweiten Endbereich (202) auf der Außenfläche des Sensorelements (20) angeordneten zweiten Kontaktfläche (44) elektrisch leitend verbunden ist, wobei die elektrisch leitende Verbindungen zwischen dem Widerstandsheizer (311) und der zweiten Kontaktflächen (44) eine im Inneren des Sensorelements (20) im Wesentlichen in Längsrichtung verlaufende zweite Leiterbahn (322) aufweist, und wobei die zweite Leiterbahn (322) und der Referenzgaskanal (35) so angeordnet sind, dass es in Aufsicht auf das Sensorelement (20) zwischen der zweiten Leiterbahn (322) und dem Referenzgaskanal (35) zu einer zweiten zumindest teilweisen Überdeckung (702) kommt und wobei die zweite Leiterbahn (322) an ihrem dem ersten Endbereich (201) des Sensorelements (20) abgewandten Ende unter einem Winkel (α) von nicht mehr als 25°, insbesondere nicht mehr als 14°, und nicht weniger als 2°, insbesondere nicht weniger als 5°, zur Außenseite des Sensorelements (20) abgewinkelt verläuft, wobei die elektrisch leitende Verbindung zwischen dem Widerstandsheizer (311) und der zweiten Kontaktfläche (44) eine zweite Durchführung (502) aufweist, die im Wesentlichen senkrecht zur Längsrichtung des Sensorelements (20) verläuft, wobei die zweite Durchführung (502) aus einer leitfähigen Beschichtung der radialen Wand eines zweiten Durchkontaktierlochs (602) des Sensorelements (20) besteht, wobei der Referenzgaskanal (35) in Aufsicht auf das Sensorelement (20) mit der zweiten Durchführung (502) überdeckungsfrei angeordnet ist, wobei das Sensorelement (20) in dem ersten Endbereich (201), im Inneren des Sensorelements (20) eine flächig ausgebildete Cermet-Elektrode (312) aufweist, die über den Referenzgaskanal (35) mit dem Außenraum des Sensorelements (20) kommuniziert, die mit einer im zweiten Endbereich (202) auf der Außenfläche des Sensorelements (20) angeordneten dritten Kontaktfläche (45) elektrisch leitend verbunden ist, wobei die elektrisch leitende Verbindung zwischen der Cermet Elektrode (312) und der dritten Kontaktfläche (45) eine im Inneren des Sensorelements (20) im Wesentlichen in Längsrichtung verlaufende dritte Leiterbahn (328) aufweist, wobei die elektrisch leitende Verbindung zwischen der Cermet Elektrode (312) und der dritten Kontaktfläche (45) neben der dritten Leiterbahn (328), mit dieser zusammenwirkend eine dritte Durchführung (503) aufweist, die im Wesentlichen senkrecht zur Längsrichtung des Sensorelements (20) verläuft, wobei die dritte Durchführung (503) aus einer leitfähigen Beschichtung der radialen Wand eines dritten Durchkontaktierlochs (603) des Sensorelements (20) besteht und der Referenzgaskanal (35) in Aufsicht auf das Sensorelement (20) mit der dritten Durchführung (503) überdeckungsfrei angeordnet ist, wobei die dritte Leiterbahn (328) und der Referenzgaskanal (35) so angeordnet sind, dass es in Aufsicht auf das Sensorelement (20) zwischen ihnen zu einer dritten zumindest teilweisen Überdeckung (703) kommt, wobei die dritte Leiterbahn (328) an ihrem dem ersten Endbereich (201) des Sensorelements (20) abgewandten Ende unter einem Winkel (α) von nicht mehr als 25°, insbesondere nicht mehr als 14° und nicht weniger als 2°, insbesondere nicht weniger als 5°, zur Außenseite des Sensorelements (20) abgewinkelt verläuft.

2. Sensorelement nach Anspruch 1, **dadurch gekennzeichnet, dass** der Referenzgaskanal (35) ungefüllt ausgebildet ist.

3. Sensorelement nach einem der Ansprüche 1 oder 2, **dadurch gekennzeichnet, dass** der elektrische Widerstandheizer (311) einen elektrischen Widerstand von maximal 30 Ohm bei 20°C aufweist.

4. Sensorelement nach einem der Ansprüche 1 bis 3, **dadurch gekennzeichnet, dass** die dritte Überdeckung (703) sich lokal in Querrichtung des Sensorelements (20) über nicht weniger als 5%, insbesondere um nicht weniger als 20%, vorzugsweise sogar um nicht weniger als 35% der lokalen Breite des Referenzgaskanals (35) und/oder der lokalen Breite der Leiterbahn (328) erstreckt.

5. Sensorelement nach Anspruch 4, **dadurch gekennzeichnet, dass** die dritte Überdeckung (703) sich lokal in Querrichtung des Sensorelements (20) über 100% der lokalen Breite des Referenzgaskanals (35) und/oder der lokalen Breite zumindest der Leiterbahn (328) erstreckt.

6. Sensorelement nach einem der Ansprüche 1 bis 5, **dadurch gekennzeichnet, dass** die erste, zweite oder dritte Leiterbahn (321, 322, 328) in dem Bereich, in dem sie abgewinkelt verläuft, und/oder in dem Bereich in dem sie in Aufsicht auf das Sensorelement eine Kante des Referenzgaskanals (901) schneidet, eine Breite aufweist, die gegenüber einem dem ersten Endbereich des Sensorelements (20) zugewandten Bereich der jeweiligen Leiterbahn (321, 322, 328) erhöht ist, insbesondere um mindestens 25%.

## Claims

1. Sensor element, in particular for detecting a physical property of a gas, in particular for detecting the concentration of a gas component or the temperature or a solid component or a liquid component of an exhaust gas from an internal combustion engine, wherein the sensor element (20) has a first end region (201) and a second end region (202) opposite one another in its longitudinal direction, wherein the sensor element (20) has in the first end region (201), in the interior of the sensor element (20), an electrical resistance heater (311), which is connected in an electrically conducting manner to a first contact area (43), arranged in the second end region (202) on the outer surface of the sensor element (20), wherein the electrically conducting connection between the resistance heater (311) and the first contact area (43) has a first printed conductor (321), running substantially in the longitudinal direction in the interior of the sensor element (20), wherein the electrically conducting connection between the resistance heater (311) and the first contact area (43) has in addition to the first printed conductor (321), interacting with it, a first lead-through (501), which runs substantially perpendicularly to the longitudinal direction of the sensor element (20), wherein the first lead-through (501) consists of a conductive coating of the radial wall of a first plated-through hole (601) of the sensor element (20), wherein the sensor element (20) also has a reference gas duct (35), running substantially in the longitudinal direction of the sensor element (20) and communicating by way of a reference gas opening (351) with a reference gas located outside the sensor element (20), wherein the reference gas duct (35) is arranged without overlapping the first lead-through (501) in plan view of the sensor element (20), wherein the first printed conductor (321) and the reference gas duct (35) are arranged such that there is between them a first at least partial overlap (701) in plan view of the sensor element (20), wherein, at its end away from the end region (201) of the sensor element (20), the first printed conductor (321) is angled away by an angle (α) of no more than 25°, in particular no more than 14°, and no less than 2°, in particular no less than 5°, in relation to the outer side of the sensor element (20), and wherein the resistance heater (311) is connected in an electrically conducting manner to a second contact area (44), arranged in the second end region (202) on the outer surface of the sensor element (20), wherein the electrically conducting connections between the resistance heater (311) and the second contact areas (44) has a second printed conductor (322), running substantially in the longitudinal direction in the interior of the sensor element (20), and wherein the second printed conductor (322) and the reference gas duct (35) are arranged such that there is between the second printed conductor (322) and the reference gas duct (35) a second at least partial overlap (702) in plan view of the sensor element (20) and wherein, at its end away from the first end region (201) of the sensor element (20), the second printed conductor (322) is angled away by an angle (α) of no more than 25°, in particular no more than 14°, and no less than 2°, in particular no less than 5°, in relation to the outer side of the sensor element (20), wherein the electrically conducting connection between the resistance heater (311) and the second contact area (44) has a second lead-through (502), which runs substantially perpendicularly to the longitudinal direction of the sensor element (20), wherein the second lead-through (502) consists of a conductive coating of the radial wall of a second plated-through hole (602) of the sensor element (20), wherein the reference gas duct (35) is arranged without overlapping the second lead-through (502) in plan view of the sensor element (20), wherein the sensor element (20) has in the first end region (201), in the interior of the sensor element (20), a two-dimensionally formed cermet electrode (312), which communicates with the exterior space of the sensor element (20) by way of the reference gas duct (35) and is connected in an electrically conducting manner to a third contact area (45), arranged in the second end region (202) on the outer surface of the sensor element (20), wherein the electrically conducting connection between the cermet electrode (312) and the third contact area (45) has a third printed conductor (328), running substantially in the longitudinal direction in the interior of the sensor element (20), wherein the electrically conducting connection between the cermet electrode (312) and the third contact area (45) has in addition to the third printed conductor (328), interacting with it, a third lead-through (503), which runs substantially perpendicularly to the longitudinal direction of the sensor element (20), wherein the third lead-through (503) consists of a conductive coating of the radial wall of a third plated-through hole (603) of the sensor element (20) and the reference gas duct (35) is arranged without overlapping the third lead-through (503) in plan view of the sensor element (20), wherein the third printed conductor (328) and the reference gas duct (35) are arranged such that there is between them a third at least partial overlap (703) in plan view of the sensor element (20), wherein, at its end away from the first end region (201) of the sensor element (20), the third printed conductor (328) is angled away by an angle (α) of no more than 25°, in particular no more than 14°, and no less than 2°, in particular no less than 5°, in relation to the outer side of the sensor element (20) .

2. Sensor element according to Claim 1, **characterized in that** the reference gas duct (35) is formed as unfilled.

3. Sensor element according to either of Claims 1 and 2, **characterized in that** the electrical resistance heater (311) has an electrical resistance of a maximum of 30 Ohms at 20°C.

4. Sensor element according to one of Claims 1 to 3, **characterized in that** third overlap (703) extends locally in the transverse direction of the sensor element (20) across no less than 5%, in particular no less than 20%, preferably even no less than 35%, of the local width of the reference gas duct (35) and/or the local width of the printed conductor (328) .

5. Sensor element according to Claim 4, **characterized in that** the third overlap (703) extends locally in the transverse direction of the sensor element (20) across 100% of the local width of the reference gas duct (35) and/or the local width at least of the printed conductor (328).

6. Sensor element according to one of Claims 1 to 5, **characterized in that** the first, second or third printed conductor (321, 322, 328) has in the region in which it is angled away and/or in the region in which it overlaps an edge of the reference gas duct (901) in plan view of the sensor element a width that is increased, in particular by at least 25%, in comparison with a region of the respective printed conductor (321, 322, 328) towards the first end region of the sensor element (20).

## Revendications

1. Élément capteur, destiné notamment à mettre en évidence une propriété physique d'un gaz, notamment pour mettre en évidence la concentration d'un composant gazeux ou la température ou un composant solide ou un composant liquide d'un gaz d'échappement d'un moteur à combustion interne, dans lequel l'élément capteur (20) comporte, dans sa direction longitudinale, une première zone d'extrémité (201) et une deuxième zone d'extrémité (202) qui sont opposées l'une à l'autre, dans lequel l'élément capteur (20) comporte, dans la première zone d'extrémité (201), à l'intérieur de l'élément capteur (20), un élément chauffant à résistance électrique (311) qui est relié de manière électriquement conductrice à une première surface de contact (43) disposée dans la deuxième zone d'extrémité (202) sur la surface externe de l'élément capteur (20), dans lequel la liaison électriquement conductrice entre l'élément chauffant à résistance (311) et la première surface de contact (43) comporte une première piste conductrice (321) s'étendant sensiblement dans la direction longitudinale à l'intérieur de l'élément capteur (20), dans lequel la liaison électriquement conductrice entre l'élément chauffant à résistance (311) et la première surface de contact (43), outre la première piste conductrice (321), comporte une première traversée (501) coopérant avec celle-ci et s'étendant sensiblement perpendiculairement à la direction longitudinale de l'élément capteur (20), dans lequel la première traversée (501) est constituée d'un revêtement conducteur de la paroi radiale d'un premier trou traversant (601) de l'élément capteur (20), dans lequel l'élément capteur (20) comprend en outre un conduit de gaz de référence (35) s'étendant sensiblement dans la direction longitudinale de l'élément capteur (20) et qui communique avec un gaz de référence se trouvant à l'extérieur de l'élément capteur (20) par l'intermédiaire d'un orifice de gaz de référence (351), dans lequel le conduit de gaz de référence (35) est disposé de telle sorte que, lorsque l'élément capteur (20) est vu de dessus, il ne chevauche pas la première traversée (501), dans lequel la première piste conductrice (321) et le conduit de gaz de référence (35) sont disposés de telle sorte que, lorsque l'élément capteur (20) est vu de dessus, il se produit au moins partiellement un premier chevauchement (701) entre eux, dans lequel la première piste conductrice (321), à son extrémité tournée à l'opposé de la première zone d'extrémité (201) de l'élément capteur (20), forme un angle (α) non supérieur à 25°, notamment non supérieur à 14°, et non inférieur à 2°, notamment non inférieur à 5°, par rapport à la face externe de l'élément capteur (20), et dans lequel l'élément chauffant à résistance (311) est relié de manière électriquement conductrice à une deuxième surface de contact (44) disposée dans la deuxième zone d'extrémité (202) sur la surface externe de l'élément capteur (20), dans lequel les liaisons électriquement conductrices entre l'élément chauffant à résistance (311) et les deuxièmes surfaces de contact (44) comporte une deuxième piste conductrice (322) s'étendant sensiblement dans la direction longitudinale à l'intérieur de l'élément capteur (20), et dans lequel la deuxième piste conductrice (322) et le conduit de gaz de référence (35) sont disposés de telle sorte que, lorsque l'élément capteur (20) est vu de dessus, il se produit au moins partiellement un deuxième chevauchement (702) entre la deuxième piste conductrice (322) et le conduit de gaz de référence (35), et dans lequel la deuxième piste conductrice (322), à son extrémité tournée à l'opposé de la première zone d'extrémité (201) de l'élément capteur (20), forme un angle (α) non supérieur à 25°, notamment non supérieur à 14°, et non inférieur à 2°, notamment non inférieur à 5°, par rapport à la face externe de l'élément capteur (20), dans lequel la liaison électriquement conductrice entre l'élément chauffant à résistance (311) et la deuxième surface de contact (44) comporte une deuxième traversée (502) s'étendant sensiblement perpendiculairement à la direction longitudinale de l'élément capteur (20), dans lequel la deuxième traversée (502) est constituée d'un revêtement conducteur de la paroi radiale d'un deuxième trou traversant (602) de l'élément capteur (20), dans lequel le conduit de gaz de référence (35) est disposé de telle sorte que, lorsque l'élément capteur (20) est vu de dessus, il ne chevauche pas la deuxième traversée (502), dans lequel l'élément capteur (20) comporte, dans la première zone d'extrémité (201), à l'intérieur de l'élément capteur (20), une électrode en cermet (312) réalisée de manière plane qui communique par l'intermédiaire du conduit de gaz de référence (35) avec l'espace externe de l'élément capteur (20) et qui est reliée de manière électriquement conductrice à une troisième surface de contact (45) disposée dans la deuxième zone d'extrémité (202) sur la surface externe de l'élément capteur (20), dans lequel la liaison électriquement conductrice entre l'électrode en cermet (312) et la troisième surface de contact (45) comporte une troisième piste conductrice (328) s'étendant sensiblement dans la direction longitudinale à l'intérieur de l'élément capteur (20), dans lequel la liaison électriquement conductrice entre l'électrode en cermet (312) et la troisième surface de contact (45), outre la troisième piste conductrice (328), comporte une troisième passage (503) coopérant avec celle-ci et s'étendant sensiblement perpendiculairement à la direction longitudinale de l'élément capteur (20), dans lequel la troisième traversée (503) est constituée d'un revêtement conducteur de la paroi radiale d'un troisième trou traversant (603) de l'élément capteur (20) et le conduit de gaz de référence (35) est disposé de telle sorte que, lorsque l'élément capteur (20) est vu de dessus, il ne chevauche pas la troisième traversée (503), dans lequel la troisième piste conductrice (328) et le conduit de gaz de référence (35) sont disposés de telle sorte que, lorsque l'élément capteur (20) est vu de dessus, il se produit au moins partiellement un troisième chevauchement (703) entre eux, dans lequel la troisième piste conductrice (328), à son extrémité tournée à l'opposé de la première zone d'extrémité (201) de l'élément capteur (20), forme un angle (α) non supérieur à 25°, notamment non supérieur à 14°, et non inférieur à 2°, notamment non inférieur à 5°, par rapport à la face externe de l'élément capteur (20).

2. Élément capteur selon la revendication 1, **caractérisé en ce que** le conduit de gaz de référence (35) n'est pas rempli.

3. Élément capteur selon l'une des revendications 1 ou 2, **caractérisé en ce que** l'élément chauffant à résistance électrique (311) présente une résistance électrique maximale de 30 ohms à 20°C.

4. Élément capteur selon l'une des revendications 1 à 3, **caractérisé en ce que** le troisième chevauchement (703) s'étend localement dans la direction transversale de l'élément capteur (20) sur au moins 5%, notamment sur au moins 20%, et de manière préférable sur au moins 35% de la largeur locale du conduit de gaz de référence (35) et/ou de la largeur locale de la piste conductrice (328).

5. Élément capteur selon la revendication 4, **caractérisé en ce que** le troisième chevauchement (703) s'étend localement dans la direction transversale de l'élément capteur (20) sur 100% de la largeur locale du conduit de gaz de référence (35) et/ou de la largeur locale d'au moins la piste conductrice (328).

6. Élément capteur selon l'une des revendications 1 à 5, **caractérisé en ce que** la première, la deuxième ou la troisième piste conductrice (321, 322, 328), dans la zone dans laquelle elle forme un certain angle, et/ou dans la zone dans laquelle elle coupe un bord du conduit de gaz de référence (901), lorsque l'élément capteur est vu de dessus, présente une largeur qui augmente par rapport à une zone de la piste conductrice (321, 322, 328) respective qui est tournée vers la première zone d'extrémité de l'élément capteur (20), notamment d'au moins 25%.
